(19) Europäisches Patentamt / European Patent Office / Office européen des brevets

(11) **EP 3 522 962 B1**

## (12) EUROPEAN PATENT SPECIFICATION

(45) Date of publication and mention
of the grant of the patent:
**02.09.2020  Bulletin 2020/36**

(21) Application number: **17777602.8**

(22) Date of filing: **06.10.2017**

(51) Int Cl.:
**A61M 15/08** $^{(2006.01)}$     **A61F 5/56** $^{(2006.01)}$

(86) International application number:
**PCT/EP2017/075504**

(87) International publication number:
**WO 2018/065588 (12.04.2018 Gazette 2018/15)**

(54) **A NASAL DELIVERY SYSTEM**

SYSTEM ZUR NASALEN VERABREICHUNG

SYSTÈME D'ADMINISTRATION NASALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.10.2016  EP 16192545**

(43) Date of publication of application:
**14.08.2019  Bulletin 2019/33**

(73) Proprietor: **B-Helpware ApS
8240 Risskov (DK)**

(72) Inventors:
• **BJERRUM, Stefan
8240 Risskov (DK)**
• **BJERRUM, Emil, Andreas, Kjeldahl
2100 Copenhagen Ø. (DK)**

(74) Representative: **Inspicos P/S
Kogle Allé 2
2970 Hørsholm (DK)**

(56) References cited:
**US-A1- 2009 248 058     US-A1- 2014 246 023
US-A1- 2016 081 845**

## Description

Field of the invention

The present invention relates to a nasal delivery system delivering a substance/fluid medium to the nasal airway. Particularly, the invention relates to a system for nasal delivery of drugs.

Background of the invention

The nasal airway forms two nasal cavities separated by a nasal septum. Different conditions can be treated by nasal administration of drugs. As an example, different allergic reactions can be treated by inhalation of suitable drugs and different vaccines can be suspended in liquid solutions and inhaled. In another example, nasal congestion leading to snoring and sleep disturbance can be treated by inhalation of suitable drugs.

Existing devices for nasal delivery often suffer from not being very precise. An adjustable nasal dilator device, insertable within the nasal cavity, is disclosed in US2014/0246023. Traditionally, nasal administration of drugs is carried out by inhalation while adding the drug by pressing a drug reservoir. Accordingly, long term delivery is not possible, and dosage can be relatively unprecise.

Description of the invention

It is an object of embodiments of the invention to increase the precision by which drugs are administered and to improve the well-being during nasal administration.

It is a further object of embodiments of the invention to provide a solution to long term administration, e.g. for administration during sleep.

According to these and other objects, the invention provides a system for nasal delivery, the system comprising a nasal insertion tube and a fluid medium in communication with the nasal insertion tube.

The nasal insertion tube comprises a tubular wall forming an internal flow path from an inlet to an outlet.

The outlet is configured to be arranged in a nostril of a person to thereby facilitate an upstream flow of air in a first flow path from an ambient space through the internal flow path to the respiratory tract of the person during inhaling and a downstream flow of air in a second flow path from the respiratory tract of the person outside the internal flow path to the ambient space during exhaling, e.g. along an outer surface of the tubular wall.

The system further comprises a flow diverter structure configured for providing a first ratio between the flow resistance in the first flow path and the flow resistance in the second flow path during upstream flow and for providing a second ratio between the flow resistance in the first flow path and the flow resistance in the second flow path during downstream flow where the second ratio is higher than or equal to the first ratio.

Due to the change in ratio for downstream and upstream flow, a higher percentage of the inhaled air will flow in the first flow path as compared with exhaling. When a higher percentage of inhaled air flows in the internal flow path, the flow speed at the entrance increases and the flow in the internal flow path thus motivates transport of the fluid medium which is in communication with the nasal insertion tube such that it can be delivered to the nasal cavity of the person using the system.

Since the flow speed in the internal flow path is higher during inhaling than during exhaling, the motivation to transport the fluid medium to the internal flow path is higher during inhaling than during exhaling. The invention may thereby provide a primary transport of the fluid medium from the system during inhaling.

Due to the higher airspeed during inhaling, the system according to the invention facilitates better control of the amount of the fluid medium which is delivered and since the flow resistance during exhaling is lower, the system offers improved comfort and eases respiration. Accordingly, the system facilitates prolonged usage, e. g. during sleep.

Herein, the ratio between the flow resistance in the first flow path and the flow resistance in the second flow path is defined as:

$$\frac{F1}{F2}$$

Where F1 is the flow resistance in the first flow path and F2 is the flow resistance in the second flow path. Accordingly, for an increasing flow resistance in the first flow path and/or for a decreasing flow resistance in the second flow path, the ratio increases, vice versa.

The change in ratio can be obtained in different ways. In one embodiment, the flow diverter structure is configured to provide a first flow resistance in the second flow path during exhaling and a second, increased, flow resistance in the second flow path during inhaling to thereby change the ratio between the flow resistance in the first flow path and the flow resistance in the second flow path.

Herein, the first flow path from the ambient space through the internal flow path to the respiratory tract means a flow path created during inhaling. This flow path can be combined with a flow of air outside the internal flow path such that only a certain percentage of the inhaled air flows through the internal flow path, e.g. 10, 20, 30, 40, 50, 60 or even more than 60 percent of the inhaled air flows through the internal flow path during inhaling.

Herein, the downstream flow of air in the second flow path is from the respiratory tract to the ambient space outside the internal flow path. Outside the internal flow path herein means that it is separated from the internal flow path and from the fluid medium which is in communication with the flow path. It could be because it flows from the nostrils along an outer surface of the tubular wall to the ambient space, or it could flow in an inner tube inside the internal flow path and therefore separated from the internal flow path.

By fluid communication between the fluid medium and the nasal insertion tube is herein meant that the fluid medium is inside the nasal insertion tube or that the fluid medium can flow into the nasal insertion tube whereby the fluid medium may subsequently flow in the nasal insertion tube.

The fluid medium may simply be contained in the internal flow path. In one embodiment, the fluid medium is a substance which is coated onto an inner surface of the insertion tube, or which is contained in cavities inside the insertion tube.

In one embodiment, the system comprises a container containing the fluid medium, and an entrance between the inlet and outlet, the entrance being in fluid communication with the container for establishing a fluid flow from the fluid container to the internal flow path. The container could be reversibly attachable to the nasal insertion tube.

Alternatively, or in combination, the ratio can be changed by the flow diverter structure configured to provide a first flow resistance in the first flow path during inhaling and a second, increased, flow resistance in the first flow path during exhaling to thereby change the ratio between the flow resistance in the first flow path and the flow resistance in the second flow path.

The system may be used for releasing air freshening fluids, e.g. liquid or a vapor of liquids having a pleasant fragrance, e.g. to prevent unpleasant odors.

The system may be used for release fluids e.g. in form of vapors. The fluid medium may e.g. cause shrinking of the nasal lining and thereby provide better air-passage. Different matters may be added to improve this process, e.g. menthol or camphor.

The system may e.g. be used at sleep where a reduced air-passage can reduce the sleeping quality and cause snoring.

The system may simply be used for release a liquid for moistening the mucous membranes. During flights and in air conditioned environments low humidity dries the mucous membranes, especially in the nose. By using the system, the nose will be continuously moistened.

In one embodiment, the flow diverter structure is arranged at the outer surface of the tubular wall.

The flow diversion structure may e.g. comprise a collar of an elastically deformable material arranged about the tubular wall and being configured to expand and contract by a change in pressure at the outer surface on opposite sides of the collar. The collar could e.g. form a radial outward flange of an elastically formable material and configured to flex back and forth under influence of an airflow in the second flow path between an expanded configuration where it blocks a relatively large part of the flow space about the tubular wall and a collapsed configuration where it blocks a relatively small part of the flow space about the tubular wall. It may e.g. change between blocking more than 50 percent and less than 25 percent.

In one embodiment, the nasal insertion tube forms a U-shape whereby the flow path forms a first flow section from the inlet to one end of an intermediate flow section and a second flow section extending essentially parallel to the first flow section from another end of the intermediate flow section to the outlet. The intermediate flow section may extend transverse to the first and second flow sections and it may form the entrance. In this embodiment, the entrance may be formed in the intermediate flow section. Both of the legs could be inserted in individual nostrils, or at least one of the legs could be inserted in one of the nostrils.

To improve the ability to control delivery of the fluid medium in the container, the system may comprise a flow control structure comprising an electrically operated processor. The processor is configured to control at least one of the air flow in the internal flow path or the flow of the fluid medium into the internal flow path. Particularly, the processor may be configured to control the flow based on a control signal, such as an electrical signal.

It should be understood, that the system in one embodiment may comprise and/or may be in communication with one or more processors. By communication with a processor should be understood, that at least some elements of the system may be controlled by control signals provided by the at least one processor.

The use of electrical signals for controlling the flow increases the options to control based on different events. In one embodiment, the electrical signal is generated by a timer or by a sensor capable of sensing a bio-signal from a person.

The timer may be used for controlling the flow depending on a point in time. This may be suitable e.g. if the fluid medium is a drug which is preferably administered by the hour, e.g. one dose four times a day, etc.

In one embodiment, a timer may be used to release medicine 1-2 hours before the person wakes up. This may be of particular importance for people with Parkinson's and rheumatoid arthritis, as they often wake up in the morning and are completely stiff in muscles and joints. As it may take one to two hours before the medicine starts to work, their mornings are often ruined. This may be counteracted by release of medicine 1-2 hours before they wake up.

A timer may also assist in medication of children with ADHD. As an example, the medicine may be given e.g. 30 minutes before they have to get up whereby they may be significantly more fit when they get up of bed. Furthermore, their parents can avoid struggling with them in order to get them to take the medicine.

The bio-signal may be used for controlling the flow depending on a bio signal sensed from the user. In one example, the bio signal may indicate if the person sleeps or is awake, or it may indicate the stage of the sleep, e.g. REM or non-REM, or if the drug is particularly designed for treating sleep affected conditions such as snoring.

The bio-signal may be obtained from the blood pressure, pulse, temperature, electrical activity, humidity of the person, or pulse oximetry. This may be suitable e. g. if the ability of the fluid medium to treat the person depends on heart-rate, blood pressure, oxygenation, etc. Accordingly, the system may include a sensor capable of sensing one of these bio-signals, and the afore-mentioned electrical signals controlling them may be generated based on a signal from the sensor.

The system may comprise an actuator arranged to move the flow diverter structure in response to commands from the processor. The actuator may be an electrically driven actuator and may e.g. include a piezo-electric element or other kinds of small mechanical actuation systems.

The system may furthermore comprise a pumping system for actively moving the fluid medium into the internal flow path.

To increase efficiency and to prevent malfunctioning due to a clotted tube, the system may comprise an insertion tube forming a double tube with an additional flow path extending in parallel with the internal flow path. In this embodiment, both flow paths may be connected to the same container to thereby deliver the same fluid medium, or they may be connected to individual containers thereby enabling one of the flow path to deliver one fluid medium and the other flow path to deliver another fluid medium. The double tube system may be arranged such that the outlet of each tube is insertable into individual nostrils.

The fluid medium may contain drugs, e.g. dissolved or as a liquid suspension. The fluid medium may also be in form of powder, and for that purpose, the system may include a blower fan to move the powder from the container to the outlet of the insertion tube. In the context of the present invention, the term "fluid" should be understood, as a gas, a liquid, a powder, or combinations hereof.

The system may include a nebulizer to provide nebulizing of the fluid medium before leaves the insertion tube and enters the nasal cavity.

The system may further include heating means, e.g. a resistive wire in the insertion tube, or in the container. The heating means enable heating of the fluid medium, e.g. for bringing it on vapour form before it is released.

Parts of the system may be pressurized to provide a pressure difference enhancing transport of the fluid medium or for releasing it in atomized form etc.

The system may comprise a converter box enabling positioning of the container at a distance from the insertion tube, e.g. at a cheek, behind the ear, or at slightly greater distance e.g. on the chest. It can also be placed on the bedside table or hanging as drop beside the bed.

The container could be designed as a box which can be used several times. In one embodiment, an elastically deformable coupling allows decoupling and coupling of the container to the system thereby allowing repeatable coupling and decoupling. Therefore, it can easily be mounted to e.g. the intermediate flow section.

In order to monitor breathing of a person using the nasal delivery system, the system may further comprise a breathing sensor configured to determine a flow in the internal flow path by measuring an electrical signal generated by the flow. As an example, the system may comprise an element arranged in the nasal insertion tube or coupled to the nasal insertion tube. At least a part of this element may move by impact of a flow in the internal flow path whereby the movement may cause the generation of an electrical signal.

In one embodiment, the breathing sensor may comprise at least one magnet movably arranged in the nasal insertion tube. The magnet may move by impact of a flow in the internal flow path. Furthermore, the breathing sensor may comprise a coil arranged around the nasal insertion tube. When the magnet moves in the tube, an electrical signal will be generated by in the coil by induction. When monitoring/measuring the induced electrical signal, breathing can be monitored/measured. An amplified may be need to achieve accurate values.

In an alternative embodiment, the breathing sensor may comprise at least three elongated metal elements extending substantially parallel from a carrier, where two of the at least three metal elements are movably arranged to allow bending hereof. The three elongated metal element may be fixed to the carrier at a first end. The opposite second end may be a free end extending substantially perpendicular from the carrier.

The elongated metal elements may be arranged at a line at equidistant distance. The metal element arranged in the middle may be non-movably attached, whereas to tow metal element on each side of the middle element may be movably attached to carrier. It should be understood that the carrier may be an isolating plate which may ensure that the three metal elements are electrically isolated from each other.

An electrical signal may be generated by the flow caused by contact between two of the at least three metal elements. As an example, a flow in the internal flow path may cause one of the movable elements bend toward the middle element. Due to contact between the bending element and the non-movable element in the middle, an electrical signal can be measured.

In one embodiment, the breathing sensor may further be configured to determine a direction of the flow based on the contact between two of the at least three metal elements, as contact between one set of element may be caused by a flow in one direction and contact between the other set of element may be caused by a flow in the other direction, where one set of elements include the non-movable element in the middle and one of the movable elements, and the other set of elements include the non-movable element in the middle and the other one of the movable elements.

The present disclosure provides a method of delivering a fluid medium in a nostril of a person by use of a system according to the first aspect.

The method comprises the step of inserting the nasal insertion tube in the nostril such that the outlet is arranged in the nostril, establishing an upstream flow of air in the first flow path by inhaling and thereby establishing a fluid flow from the fluid container of the fluid medium to the flow path, establishing a downstream fluid flow in the second flow path by exhaling and moving the flow diverter structure to increase the ratio between the flow resistance in the first flow path and the flow resistance in the second flow path.

The flow diverter structure could be moved by use of the downstream fluid flow, or the flow diverter structure could be moved by use of an actuator, such as an electrically driven.

The nasal delivery system according to the invention is very suitable for performing the method steps according to the present disclosure. The remarks set forth above in relation to the system are therefore equally applicable in relation to the method.

Brief description of the drawings

Embodiments of the invention will now be further described with reference to the drawings, in which:

Fig. 1 illustrates a nasal delivery system according to the invention;

Figs. 2A-2C illustrate the nasal delivery system in Fig. 1 when inserted into nostrils of a person;

Figs. 3A-3C illustrate an embodiment of a nasal delivery system;

Figs. 4-9 illustrate different flow situations obtained with a nasal delivery system according to the invention;

Fig. 10 illustrates an embodiment with multiple insertion tubes;

Figs. 11-12 illustrate an embodiment with a container attached to a mounting surface;

Fig. 13 illustrates an embodiment with a box inserted between the insertion tube and the container;

Fig. 14 illustrates an embodiment where the box is utilised for connection of an external container;

Fig. 15 illustrates an embodiment of a nasal delivery system with an electrical sensor;

Figs. 16-17 illustrate an embodiment where the exhalation is through an inner tube in the internal flow path;

Figs. 18A and 18B illustrate an embodiment with a movable closing member;

Fig. 19 illustrates an embodiment coupled to an oxygen breathing apparatus;

Figs. 20-23 illustrate embodiments comprising one more magnets;

Fig. 24 illustrates an embodiment comprising a sensor;

Fig. 25 illustrates details of the sensor illustrated in Fig. 24;

Fig. 26 illustrates an embodiment with a container and a sensor; and

Fig. 27 illustrates different views of the embodiment illustrated in Fig. 26.

Detailed description of the drawings

It should be understood that the detailed description and specific examples, while indicating embodiments of the invention, are given by way of illustration only, since various changes and modifications within the scope of the invention will become apparent to those skilled in the art from this detailed description.

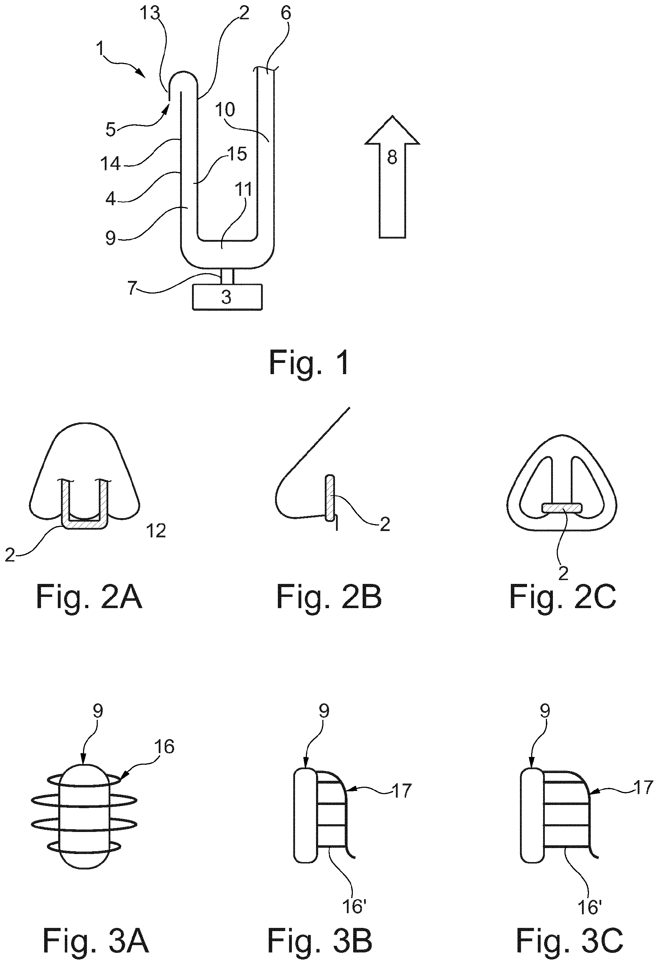

Fig. 1 illustrates a nasal delivery system 1 comprising a nasal insertion tube 2 and a container 3. The container forms space for a fluid medium, e.g. a drug solution, and it is fixed to an outer surface of the insertion tube 2 such that the container and the tube forms one integrated component. In one embodiment, the container is releasably attached to allow replacement of an empty container with a new container, and in another embodiment, the container is non-replaceable and the integrated component is to be disposed when the container is empty.

The nasal insertion tube comprises a tubular wall 4 forming an internal flow path from an inlet 5 to an outlet 6. Between the inlet and the outlet, the insertion tube comprises an entrance 7. The entrance allows the fluid medium from the container to enter the internal flow path.

In the illustrated embodiment, the nasal insertion tube is U-shaped and forms first and second legs 9, 10 connected by an intermediate leg 11. During use, the nasal insertion tube is inserted into the nostrils in the direction indicated by the arrow 8 such that each nostril receives one of the two legs 9, 10.

When the nasal insertion tube 2 is in place, the person can inhale and thereby establish an upstream flow of air through a first flow path. The first flow path extends from ambient space 12 through the internal flow path to the respiratory tract of the person.

The person can exhale and establish a downstream flow of air through a second flow path extending from the respiratory tract of the person along an outer surface of the tubular wall. Due to the downstream flow, the flow diverter structure 13 at the outer surface 14 folds down and blocks the internal flow path 15. Accordingly, the flow diverter structure provides a first flow resistance in the second flow path during exhaling and a second, increased, flow resistance in the second flow path during inhaling.

Fig. 2 illustrates the nasal delivery system 1 inserted in the nostrils of a person.

Fig. 3 illustrates embodiments of the system. Illustration A shows one leg-part 9 of a nasal insertion tube with rings 16 of silicone or similar soft elastically rubber etc. The rings facilitate retaining of the nasal insertion tube in the nostril of a person.

Illustration B shows another embodiment where the rings of silicone are replaced by vanes 16' extending outwards from the leg-part 9 of the nasal insertion tube. The vanes are complemented by a holding member 17 fixed to the radial outer ends of each vane and facilitating compression of the vanes by the user gripping the leg-part and the holding member 17 between the thumb and forefinger. In illustration B, the holding member 17 and the vanes are compressed and the system is ready for insertion. In illustration C, the holding member is released and by the elasticity of the vanes, the system expands and thereby facilitates retention in the nostril of the person.

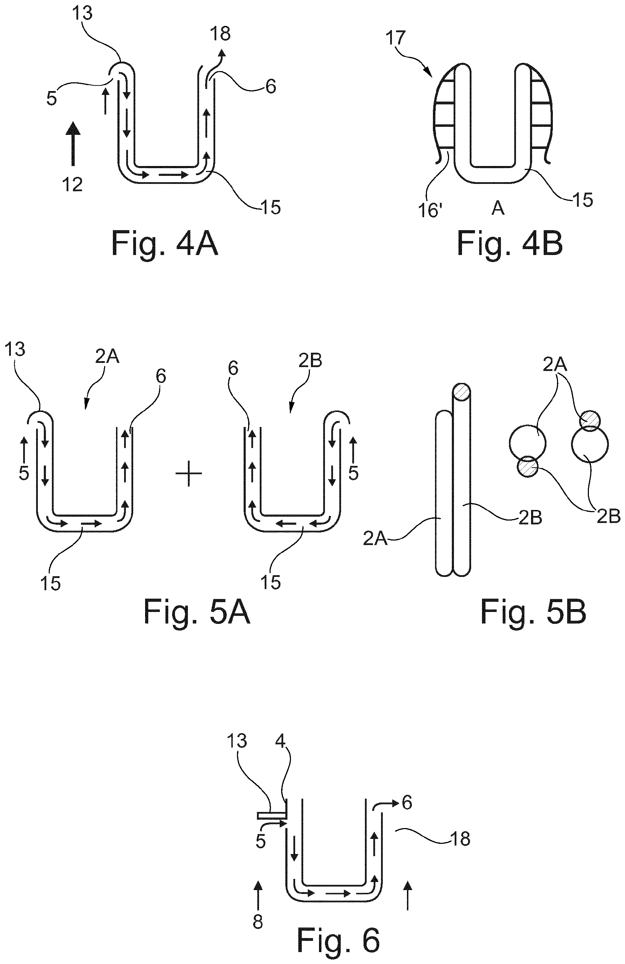

Fig. 4A illustrates with arrows, an upstream flow of air in a first flow path from an ambient space 12 through the internal flow path to the respiratory tract 18 of the person during inhaling. In the upstream flow, the flow diverter structure 13 folds out and guides the air through the internal flow path. Accordingly, the flow resistance in the first flow path is low which triggers a relatively large flow through the internal flow path.

Fig. 4B illustrates the version of Fig. 3, illustration B, C, including elastic vanes 16' and holding means for insertion into both nostrils of a person. Due to the elasticity, the holding means and the insertion tube expand the nostrils and can reduce snoring.

Fig. 5 illustrates schematically two tubes 2 in a mirrored mutual position. In this embodiment the nasal insertion tube forms a double tube with an additional flow path. As illustrated, each section of the flow path extends in parallel with the same section of the additional flow path. The two tubes of the double tube are joined e.g. by use of a resilient rubber of silicone connector (not illustrated).

In use, the outlet 6 of the flow path is placed in one nostril and the outlet 6 of the additional flow path is placed in the other nostril. The advantage of this embodiment is, that the breathing is allowed to follow a natural cycle in which one nostril is dominant for a few hours after which the other nostril takes over, vice versa. The double tube enables a more stable and homogenous intake of the fluid medium.

Figs. 6-7 illustrate an embodiment where the flow diverter structure 13 is in the form of a movable valve element.

Fig. 6 illustrates the upstream flow of air, indicated by the arrow 8. The flow diverter structure 13 is moved to a position where it seals between the outer surface of the tubular wall 4 and the inner wall of the nostril into which the left leg is inserted. The flow diverter structure therefore blocks a flow between the outer wall and the wall of the nostril and thereby facilitates a flow in the first flow path during inhaling.

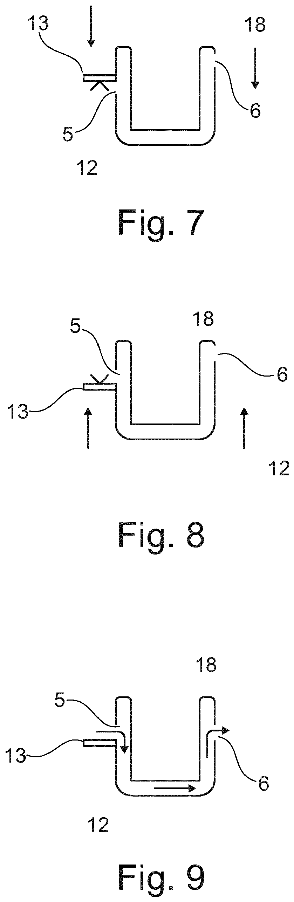

Fig. 7 illustrates that the flow diverter structure 13 forms an open valve during exhaling. The downstream flow of air is allowed to pass the insertion tube between the outer wall of the tube and the inner wall of the nostril, and there is no motivation for the air to flow through the internal flow path.

Figs. 8-9 illustrate another embodiment where the flow diverter structure is arranged in another position relative to the inlet. The flow diverter structure is arranged such that a downstream flow of air reaches the inlet before reaching the flow diverter structure. In this way, the inhaling does not provide a flow through the internal flow path. In Fig. 8, the valve is open and in Fig. 9, the valve is closed thereby the air flow is forced through the internal flow path. This embodiment may be relevant in combination with drugs which must be released during exhaling.

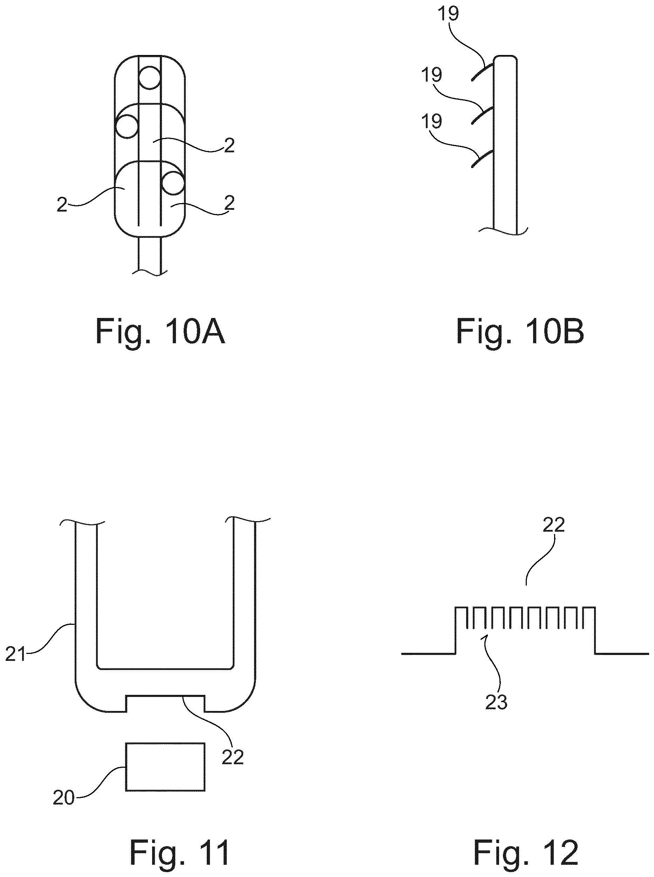

Fig. 10A illustrates a side view of an alternative embodiment of the delivery system. The delivery system comprises three joined insertion tubes. There are therefore three internal flow paths in which air can flow. Fig. 10B illustrates three flow diverters 19 corresponding to each of the three insertion tubes to improve the air intake in the internal flow path.

To facilitate manufacturing, the U-shaped tubes and the container with the fluid medium, e.g. a liquid oil or medicine, is divided into two separate units 20, 21, as illustrated in Fig. 11. They may be assembled during manufacturing, or they may be delivered separately to be assembled by the user.

The figure shows the U-shaped tube which is made with a mounting surface 22 where the container 20 can be attached.

Fig. 12 illustrates an enlarged view of the mounting surface including a plurality of small pointed tubes 23. When the container 20 is pressed against the mounting surface 22 a foil layer of the container 20 is perforated and the fluid medium will flow through the pointed tubes 23 thereby constituting the entrance.

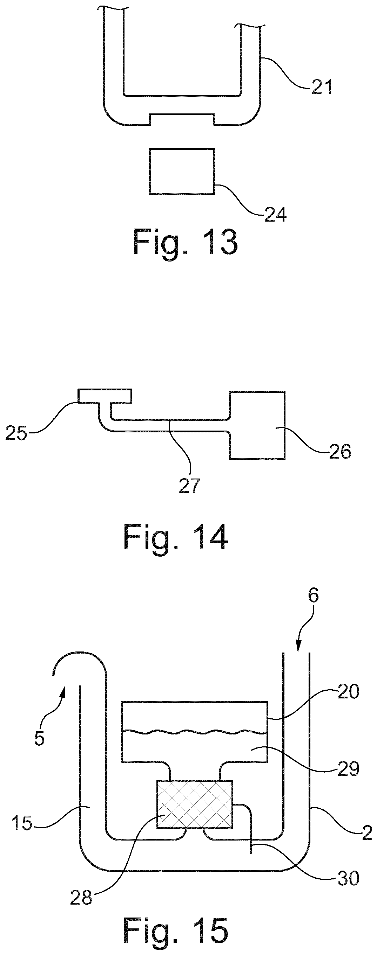

Fig. 13 illustrates an attachment box 24. The box may be configured for transport or to convert the content of the container to facilitate absorption in the nasal cavity. The box can be fitted to the mounting surface in the same way as the above described container 20. The box may have different functions. It may e.g. incorporate electrical control means, e.g. a processor and an electrically controlled valve for controlling release of the fluid medium e.g. based on a sensed bio-signal or based on temperature, or based on a clock/timer.

Fig. 14 illustrates a box 25 having the function of connecting a distal container 26 to the insertion tube via a connection pipe 27. This may allow placement of the container e.g. on the chest or on a table next to the bed.

The system may comprise a flow control structure including a sensor, e.g. a sensor which is active during the inhalation and which communicates with a processor which controls the release of the fluid medium from the container. This enables the release of the fluid medium based on a sensed property. The sensor may be located in the insertion tube, or in the box, or in the container, or in the pipe. Alternatively, the sensor may be located distant from the system, e.g. on the person. In that case, communication with the processor may be wireless or wired.

The flow control structure may e.g. effect fluid flow from the container only in response to different redefined events, e.g. at specific points in time, e.g. only during sleep, e.g. only during REM sleep.

The box can also be closed and only open if there is a sign of incipient seizure, e.g. upon detection of an incipient heart attack.

The fluid medium may be a drug substance contained as flowable powder or in liquid form. The system may include means enhancing evaporation, mixing and delivery of the substance, e.g. including a mixer, a blower fan, and heating means etc. Such elements may be placed in the box 24, in the container 3, in the pipe 27, or even in the insertion tube 2.

Fig. 15 illustrates an electronic module 28 inserted between the insertion tube 2 and the container 20. The electronic module 28 controls the flow of the fluid medium 29 from the container to the probe 30 and thus the delivery of the fluid medium in the internal flow path.

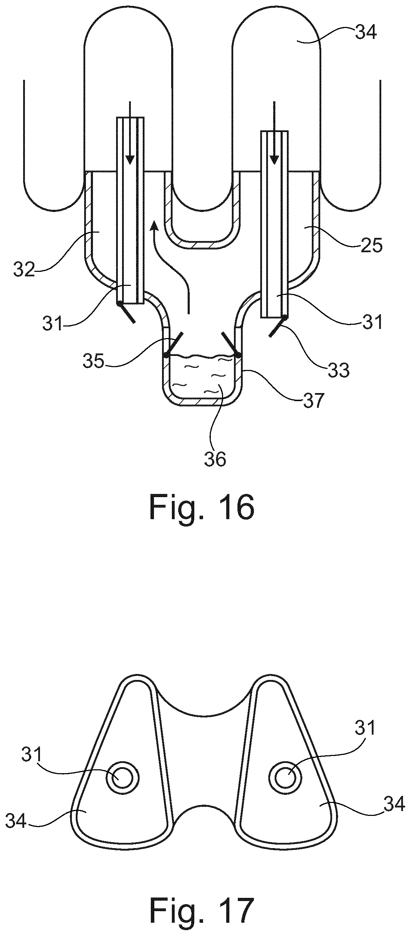

Fig. 16 illustrates an embodiment of the nasal delivery system in which the exhaust air does not flow along the outer surface of the tubular wall but rather in an inner tube 31 which extends in the internal flow path 32.

During inhaling, the air flows through the one-way valve 33 into the internal flow path 32 and into the nostrils 34. The valve 35 controls the flow of the liquid 36 from the container 37 into the internal flow path.

During exhaling, the air flows through the inner tubes 31 to the ambient space.

Fig. 17 illustrates the nasal delivery system from Fig. 16 seen from below the nostrils.

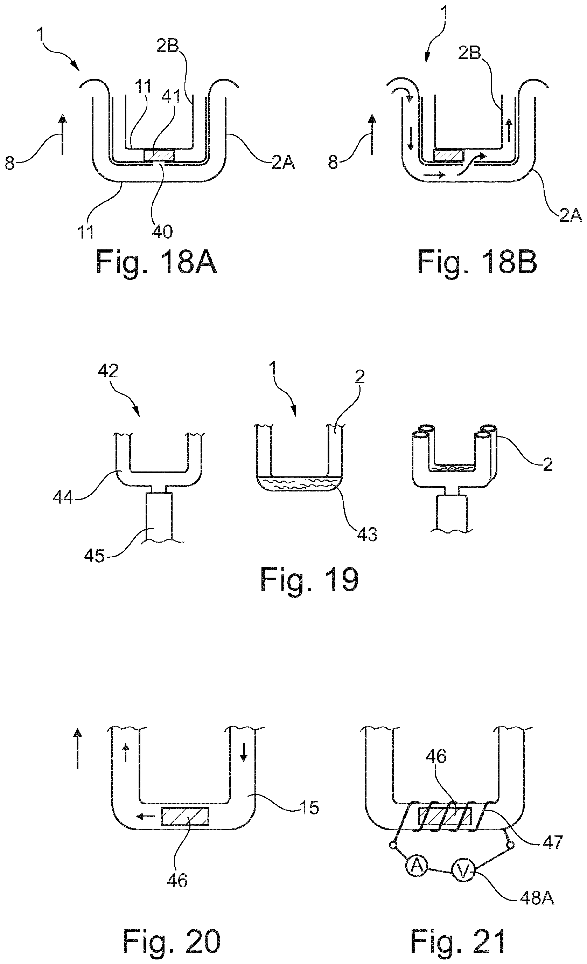

Figs. 18A and 18B illustrate an embodiment of a nasal delivery system 1 comprising two U-shaped tubes 2A, 2B. At the intermediate leg 11, both tubes form an opening 40 whereby internal flow path of the two tubes are in communication with each other. In inner tube 2B comprises a movable closing member 41. During inhalation through the left nostril (illustrated by the arrow 8), the closing member 41 will move to the left due to the open upper end, whereby the left part of the inner tube 2B is blocked and the primary flow will be from the outer tube 2A to the inner tube 2B as indicated by the arrows. Thus, the flow will be toward the non-active nostril.

The nostril with the greatest flow through is typically where the mucosa is most shrunk whereby the absorption through the mucosa may be significantly reduced. Therefore, it may be an advantage to have the flow to the nostril that is least active. However, in other cases where the medicine should be absorbed through the odour organ, it may be easier to get the medicine if the mucosa is shrunk.

By use of the closing member, it is therefore possible to control the fluid flow of powder and/or medicine in the active nostrils or in the passive nostrils depending on which model is chosen.

The movable closing member may be used in combination with a timer, whereby the nasal delivery system may be inactive until it is activated by the timer. Thus, the movable closing member 41 can be activated at the first inhalation, block the flow path with the highest flow, after which the flow will be sent in the opposite direction.

Fig. 19 illustrates an embodiment of a nasal delivery system 1 coupled to an oxygen breathing apparatus 42. During oxygen treatment the mucosa in the nose may dry out and may even chap. However, this may be avoided by moistening the oxygen to be inhaled. In one embodiment, the nasal delivery system 1 comprise two U-shaped tubes, a tube 2 comprising salt water 43 being coupled to a U-shaped tube 44 which is coupled to an oxygen delivery system 45. The two tubes are attached to each other, without communication there between. The tube 2 will emit moisture to the nostril which is active and to thereby avoid drying, while oxygen is delivered through the tube 44. The double U-shaped tube may be replaced every day to avoid infections and avoid drying while delivering oxygen into the nostril that is active.

Figs. 20-23 illustrate embodiments of a nasal delivery system comprising one or more magnets 46. In Fig. 20, a single magnet 46 is arranged in the internal flow path 15. During breathing the magnet 46 will move with the flow caused by breathing. In Fig. 21, a coil 47 is arranged around the nasal insertion tube 2, whereby it is possible to measure movement of the magnet 46 and thereby monitor the breathing. To facilitate monitoring an amplifier A is used. The electrical signal generated by movement of the magnet 46 in the coil 46 is measured by the sensor 48A.

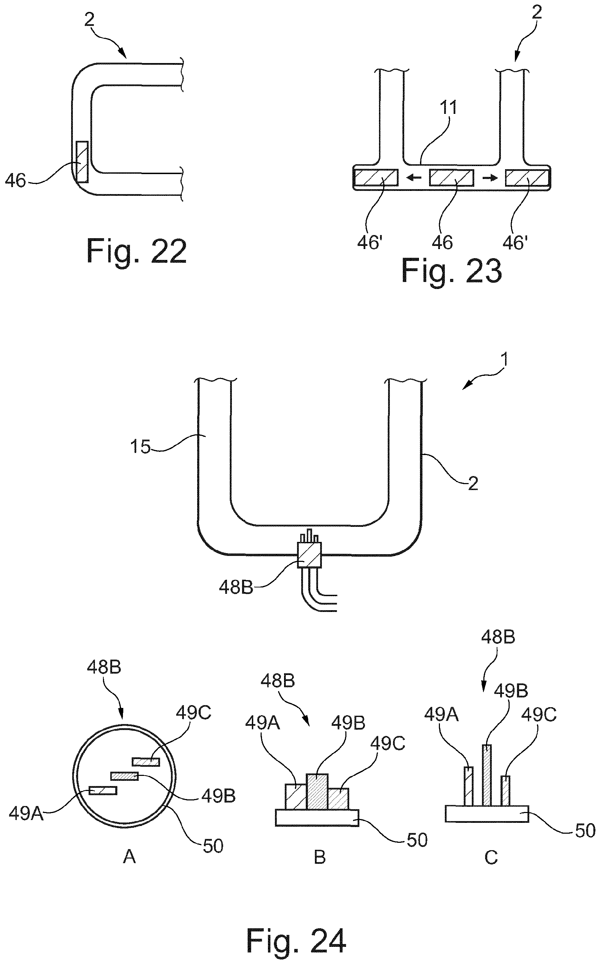

Fig. 22 illustrates that monitoring of breathing can be unreliable if the person is lying at one side (indicated by the direction of the U-shaped tube 2), as the magnet 46 will move towards the bottom in this situation. Breathing will in most situations not be able to counteract gravity, and monitoring will impossible, or at least unreliable.

If a magnet 46' is fixed at opposite ends of the intermediate leg 11, as illustrated in Fig. 23, the middle magnet 46 will move in a magnetic field whereby gravity can be neglected. If a coil is arranged around the tube as illustrated in Fig. 21, movement of the magnet 46 can be measured thereby allowing monitoring and measuring of the breathing.

Fig. 24 illustrates an embodiment of a nasal delivery system 1 comprising a measuring sensor 48B. The sensor 48B is arranged partly in the internal flow path 15 and is configured for measuring of the breathing. The sensor 48B comprises three metal elements 49A, 49B, 49C, where 49A and 49B are movably attached to the isolating back plate 50. The isolating back plate 50 ensures that the three metal elements are isolated from each other.

In Fig. 24A the sensor 48 is seen form above, whereas Figs. 24B and 24C are side view from different angles.

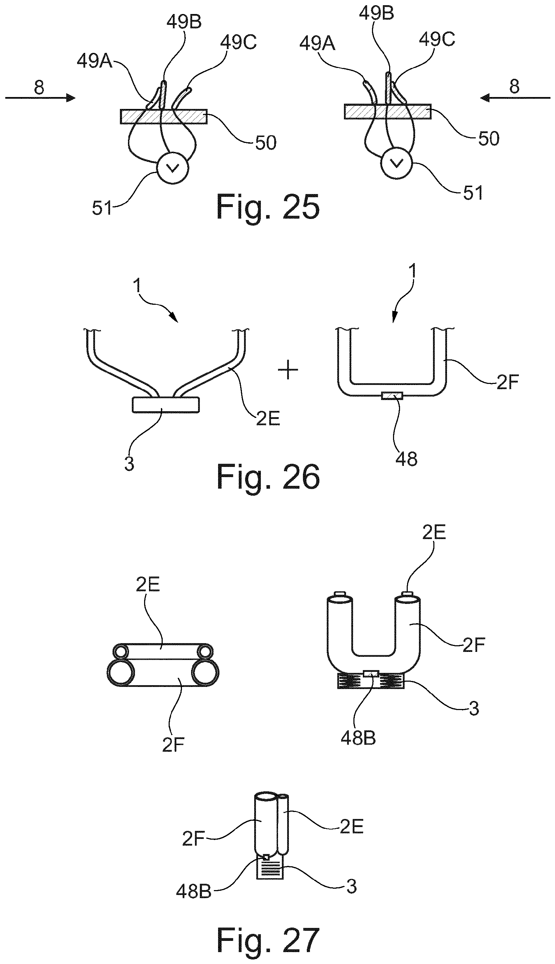

Fig. 25 illustrates details of the measuring sensor 48B of Fig. 24. In the left part of Fig. 25, the flow is toward the right side (indicated by the arrow 8). Consequently, the movable plates 49A and 39B will bend to the right. Due to contact between the element 49A and the non-movable element 49B, an electrical signal can be measured. This can be measured by the sensor 51. The right part of Fig. 25 illustrates the opposite situation where the flow is toward the left side (indicated by the arrow 8).

Fig. 26 illustrates an embodiment with a container 3 and a sensor 48. The left part of Fig. 26 illustrates a nasal delivery system 1 comprising a container 3 (as described above), whereas the right part of Fig. 26 illustrates a nasal delivery system 1 comprising a sensor 48B configured to monitor/measure breathing (as described above). The "+" indicates that the two embodiments may be combined.

Thus, medicine may be supplied from a container 3. The flow may be directed to nostril with the highest flow, e.g. by use of the movable closing member 41 as illustrated in Figs. 18A and 18B. However, by using the information from the sensor 48B which measures the breathing it will be possible to direct the medicine into the nostril with the highest flow in or in the nostril with the lowest.

It may be possible to release the medicine at the end of the tube, e.g. via a nebulizer or by a pump.

Fig. 27 illustrates different views of the embodiment illustrated in Fig. 26. The upper left part illustrates the integrated nasal delivery system 1 from above, whereas the upper right part is a front view, and the lower part is a side view.

## Claims

1. A nasal delivery system (1) comprising a nasal insertion tube (2) and a fluid medium located in communication with the nasal insertion tube (2), the nasal insertion tube (2) comprising a tubular wall (4) forming an internal flow path from an inlet (5) to an outlet (6), wherein the outlet is configured to be arranged in a nostril (34) of a person to thereby facilitate an upstream flow of air in a first flow path from an ambient space through the internal flow path to the respiratory tract of the person during inhaling and a downstream flow of air in a second flow path from the respiratory tract of the person outside the internal flow path to the ambient space during exhaling, the system further comprising a flow diverter structure (13), **characterised in that** the flow diverter structure is configured for providing a first ratio between the flow resistance in the first flow path and the flow resistance in the second flow path during upstream flow and for providing a second ratio between the flow resistance in the first flow path and the flow resistance in the second flow path during downstream flow, where the second ratio is equal to or higher than the first ratio.

2. A nasal delivery system according to claim 1, further comprising a container (3) containing the fluid medium, and an entrance (7) between the inlet and outlet, the entrance being in fluid communication with the container for establishing a fluid flow from the fluid container to the internal flow path.

3. A system according to claim 2, wherein the container is reversibly attachable to the nasal insertion tube.

4. A nasal delivery system according to any of the preceding claims, where the flow diverter structure is configured to provide a first flow resistance in the second flow path during exhaling and a second, increased, flow resistance in the second flow path during inhaling.

5. A nasal delivery system according to any of the preceding claims, where the flow diverter structure is configured to provide a first flow resistance in the first flow path during inhaling and a second, increased, flow resistance in the first flow path during exhaling.

6. A nasal delivery system according to any of the preceding claims, where the flow diverter structure is arranged at the outer surface of the tubular wall.

7. A system according to any of the preceding claims, wherein the flow diversion structure comprises a collar of an elastically deformable material arranged about the tubular wall and being configured to expand and contract by a change in pressure at the outer surface on opposite sides of the collar or by airflow in the second flow path.

**8.** A system according to claim 7, wherein the collar expands radially away from the tubular wall upon airflow towards the inlet.

**9.** A system according to any of the preceding claims, wherein the nasal insertion tube forms a U-shape whereby the flow path forms a first flow section from the inlet to one end of an intermediate flow section and a second flow section extending essentially parallel to the first flow section from another end of the intermediate flow section to the outlet, the intermediate flow section extending transverse to the first and second flow sections and forming the entrance.

**10.** A system according to any of the preceding claims, further comprising a flow control structure configured to control at least one of an air flow in the internal flow path or a flow of the fluid medium into the internal flow path, the control structure comprising a processor configured to control the flow based on a control signal.

**11.** A system according to claim 10, wherein the electrical signal is generated by a timer or by a sensor capable of sensing a bio-signal from a person.

**12.** A system according to claim 10 or 11, further comprising an actuator arranged to move the flow diverter structure.

**13.** A system according to any of the preceding claims, further comprising a breathing sensor configured to determine a flow in the internal flow path.

**14.** A system according to claim 13, wherein the flow is determined by measuring an electrical signal generated by the flow.

**15.** A system according to claim 13 or 14, wherein the breathing sensor comprises at least one magnet movably arranged in the nasal insertion tube.

**Patentansprüche**

**1.** System zur nasalen Abgabe (1), umfassend einen Naseneinführschlauch (2) und ein Fluidmedium, das in Verbindung mit dem Naseneinführschlauch (2) angeordnet ist, wobei der Naseneinführschlauch (2) eine röhrenförmige Wand (4) umfasst, die einen inneren Strömungsweg von einem Einlass (5) zu einem Auslass (6) bildet, wobei der Auslass so konfiguriert ist, dass er in einem Nasenloch (34) einer Person angeordnet ist, um dadurch einen stromaufwärtigen Luftstrom in einem ersten Strömungsweg von einem Umgebungsraum durch den inneren Strömungsweg zu den Atemwegen der Person während des Einatmens und einen stromabwärtigen Luftstrom in einem zweiten Strömungsweg von den Atemwegen der Person außerhalb des inneren Strömungswegs zum Umgebungsraum beim Ausatmen zu erleichtern, wobei das System ferner eine Strömungsumlenkerstruktur (13) umfasst, **dadurch gekennzeichnet, dass** die Strömungsumlenkerstruktur konfiguriert ist, um ein erstes Verhältnis zwischen dem Strömungswiderstand im ersten Strömungsweg und dem Strömungswiderstand im zweiten Strömungsweg während der stromaufwärtigen Strömung bereitzustellen und um ein zweites Verhältnis zwischen dem Strömungswiderstand im ersten Strömungsweg und dem Strömungswiderstand im zweiten Strömungsweg während der stromabwärtigen Strömung bereitzustellen, wobei das zweite Verhältnis gleich oder höher als das erste Verhältnis ist.

**2.** System zur nasalen Abgabe nach Anspruch 1, ferner umfassend einen Behälter (3), der das Fluidmedium enthält, und einen Eingang (7) zwischen dem Einlass und dem Auslass, wobei der Eingang in Fluidverbindung mit dem Behälter steht, um einen Fluidstrom aus dem Fluidbehälter zum internen Strömungsweg herzustellen.

**3.** System zur nasalen Abgabe nach Anspruch 2, wobei der Behälter reversibel an dem Naseneinführschlauch anbringbar ist.

**4.** System zur nasalen Abgabe nach einem der vorstehenden Ansprüche, wobei die Strömungsumlenkerstruktur konfiguriert ist, um einen ersten Strömungswiderstand im zweiten Strömungsweg während des Ausatmens und einen zweiten erhöhten Strömungswiderstand im zweiten Strömungsweg während des Einatmens bereitzustellen.

**5.** System zur nasalen Abgabe nach einem der vorstehenden Ansprüche, wobei die Strömungsumlenkerstruktur konfiguriert ist, um einen ersten Strömungswiderstand im ersten Strömungsweg während des Einatmens und einen zweiten erhöhten Strömungswiderstand im ersten Strömungsweg während des Ausatmens bereitzustellen.

**6.** System zur nasalen Abgabe nach einem der vorstehenden Ansprüche, wobei die Strömungsumlenkerstruktur an der Außenfläche der Schlauchwand angeordnet ist.

**7.** System zur nasalen Abgabe nach einem der vorstehenden Ansprüche, wobei die Strömungsumleitungsstruktur einen Kragen aus einem elastisch verformbaren Material umfasst, der um die röhrenförmige Wand angeordnet ist und so konfiguriert ist, dass er sich durch eine Druckänderung an der Außenfläche auf gegenüberliegenden Seiten des Kragens oder durch Luftströmung im zweiten Strömungsweg ausdehnt und zusammenzieht.

8. System nach Anspruch 7, wobei sich der Kragen beim Luftstrom zum Einlass radial von der Schlauchwand weg ausdehnt.

9. System nach einem der vorstehenden Ansprüche, wobei der Naseneinführschlauch eine U-Form bildet, wobei der Strömungsweg einen ersten Strömungsabschnitt vom Einlass zu einem Ende eines Zwischenströmungsabschnitts und einen zweiten Strömungsabschnitt bildet, der sich im Wesentlichen parallel zu dem ersten Strömungsabschnitt von einem anderen Ende des Zwischenströmungsabschnitts zum Auslass erstreckt, wobei sich der Zwischenströmungsabschnitt quer zum ersten und zweiten Strömungsabschnitt erstreckt und den Eingang bildet.

10. System nach einem der vorstehenden Ansprüche, ferner umfassend eine Strömungssteuerungsstruktur, die konfiguriert ist, um mindestens einen Luftstrom in dem inneren Strömungsweg oder eine Strömung des Fluidmediums in den inneren Strömungsweg zu steuern, wobei die Steuerstruktur einen Prozessor umfasst, der konfiguriert ist, um die Strömung basierend auf einem Steuersignal zu steuern.

11. System nach Anspruch 10, wobei das elektrische Signal von einem Zeitgeber oder von einem Sensor erzeugt wird, der ein Biosignal von einer Person erfassen kann.

12. System nach Anspruch 10 oder 11, ferner umfassend einen Aktuator, der angeordnet ist, um die Strömungsumlenkerstruktur zu bewegen.

13. System nach einem der vorhergehenden Ansprüche, ferner umfassend einen Atmungssensor, der konfiguriert ist, um eine Strömung in dem internen Strömungsweg zu bestimmen.

14. System nach Anspruch 13, wobei die Strömung durch Messen eines durch die Strömung erzeugten elektrischen Signals bestimmt wird.

15. System nach Anspruch 13 oder 14, wobei der Atemsensor mindestens einen Magneten umfasst, der beweglich in dem Naseneinführschlauch angeordnet ist.

**Revendications**

1. Système d'administration nasale (1) comprenant un tube d'insertion nasale (2) et un milieu fluide situé en communication avec le tube d'insertion nasale (2), le tube d'insertion nasale (2) comprenant une paroi tubulaire (4) formant une voie d'écoulement interne à partir d'une entrée (5) vers une sortie (6), dans lequel la sortie est conçue pour être agencée dans une narine (34) d'une personne pour ainsi faciliter un écoulement en amont d'air dans une première voie d'écoulement à partir d'un espace ambiant à travers la voie d'écoulement interne vers le tractus respiratoire de la personne pendant l'inhalation et un écoulement en aval d'air dans une deuxième voie d'écoulement à partir du tractus respiratoire de la personne en dehors de la voie d'écoulement interne vers l'espace ambiant pendant l'expiration, le système comprenant en outre une structure de déviation d'écoulement (13), **caractérisé en ce que** la structure de déviation d'écoulement est conçue pour fournir un premier rapport entre la résistance d'écoulement dans la première voie d'écoulement et la résistance d'écoulement dans la deuxième voie d'écoulement pendant l'écoulement en amont et pour fournir un deuxième rapport entre la résistance d'écoulement dans la première voie d'écoulement et la résistance d'écoulement dans la deuxième voie d'écoulement pendant l'écoulement en aval, où le deuxième rapport est supérieur ou égal au premier apport.

2. Système d'administration nasale (1) selon la revendication 1, comprenant en outre un récipient (3) contenant le milieu fluide, et un accès (7) situé entre l'entrée et la sortie, l'accès étant en communication fluidique avec le récipient pour l'établissement d'un écoulement de fluide à partir du récipient de fluide vers la voie d'écoulement interne.

3. Système selon la revendication 2, dans lequel le récipient peut être attaché de manière réversible au tube d'insertion nasale.

4. Système selon l'une quelconque des revendications précédentes, dans lequel la structure de déviation d'écoulement est conçue pour fournir une première résistance d'écoulement dans la deuxième voie d'écoulement pendant l'expiration et une deuxième résistance d'écoulement, accrue, dans la deuxième voie d'écoulement pendant l'inhalation.

5. Système selon l'une quelconque des revendications précédentes, dans lequel la structure de déviation d'écoulement est conçue pour fournir une première résistance d'écoulement dans la première voie d'écoulement pendant l'inhalation et une deuxième résistance d'écoulement, accrue, dans la première voie d'écoulement pendant l'expiration.

6. Système selon l'une quelconque des revendications précédentes, dans lequel la structure de déviation d'écoulement est agencée au niveau de la surface externe de la paroi tubulaire.

**7.** Système selon l'une quelconque des revendications précédentes, dans lequel la structure de déviation d'écoulement comprend un collier constitué d'un matériau élastiquement déformable agencé autour de la paroi tubulaire et étant agencé pour s'étendre et se contracter à la suite d'un changement de pression au niveau de la surface extérieure sur les parois opposées du collier ou à la suite d'un écoulement d'air dans la deuxième voie d'écoulement.

**8.** Système selon la revendication 7, dans lequel le collier s'étend de manière radiale en s'éloignant de la paroi tubulaire lors de l'écoulement d'air vers l'entrée.

**9.** Système selon l'une quelconque des revendications précédentes, dans lequel le tube d'insertion nasale est en forme de U moyennant quoi la voie d'écoulement forme une première section d'écoulement provenant de l'entrée vers une extrémité d'une section d'écoulement intermédiaire et une deuxième section d'écoulement s'étendant sensiblement de manière parallèle à la première section d'écoulement à partir d'une extrémité de la section d'écoulement intermédiaire vers la sortie, la section d'écoulement intermédiaire s'étendant de manière transversale par rapport à la première et à la deuxième section d'écoulement et formant l'accès.

**10.** Système selon l'une quelconque des revendications précédentes, comprenant en outre une structure de commande d'écoulement conçu pour contrôler au moins l'un parmi un écoulement d'air dans la voie d'écoulement interne ou un écoulement du milieu fluide dans la voie d'écoulement interne, la structure de commande comprenant un processeur conçu pour contrôler l'écoulement en fonction d'un signal de commande.

**11.** Système selon la revendication 10, dans lequel le signal électrique est généré par une minuterie ou par un capteur capable de détecter un bio-signal provenant d'une personne.

**12.** Système selon la revendication 10 ou la revendication 11, comprenant en outre un actionneur agencé pour déplacer la structure de déviation d'écoulement.

**13.** Système selon l'une quelconque des revendications précédentes, comprenant en outre un capteur de respiration conçu pour déterminer un écoulement dans la voie d'écoulement interne.

**14.** Système selon la revendication 13, dans lequel l'écoulement est déterminé par la mesure d'un signal électrique généré par l'écoulement.

**15.** Système selon la revendication 13 ou la revendication 14, dans lequel le capteur de respiration comprend au moins un aimant agencé de manière amovible dans le tube d'insertion nasale.

Fig. 1

Fig. 2A

Fig. 2B

Fig. 2C

Fig. 3A

Fig. 3B

Fig. 3C

Fig. 4A

Fig. 4B

Fig. 5A

Fig. 5B

Fig. 6

Fig. 7

Fig. 8

Fig. 9

Fig. 10A

Fig. 10B

Fig. 11

Fig. 12

Fig. 13

Fig. 14

Fig. 15

Fig. 16

Fig. 17

Fig. 18A

Fig. 18B

Fig. 19

Fig. 20

Fig. 21

Fig. 22

Fig. 23

Fig. 24

**Fig. 25**

**Fig. 26**

**Fig. 27**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140246023 A